# EUROPÄISCHE PATENTANMELDUNG

(11) **EP 1 355 159 A1**
(43) Veröffentlichungstag der Anmeldung: **22.10.2003**
(21) Anmeldenummer: 02008841.5
(22) Anmeldetag: 19.04.2002
(51) Int. Cl.: G01N 33/68, G01N 33/569, G01N 33/573

(54) **Verwendungen von Fragmenten der Carbamoylphosphat Synthetase 1 (CPS 1) für die Diagnose von Entzündungserkrankungen und Sepsis**

(71) Anmelder: B.R.A.H.M.S Aktiengesellschaft, 16761 Hennigsdorf (DE)
(72) Erfinder: Bergmann, Andreas, Dr., 12351 Berlin (DE); Struck, Joachim, Dr., 12161 Berlin (DE); Ühlein, Monika, Dr., 10407 Berlin (DE)
(74) Vertreter: Andrae, Steffen, Dr.

(57) **Zusammenfassung**

Verwendung von Fragmenten des N-terminalen Teils der Carbamoylphosphat Synthetase (CPS 1) aus Körperflüssigkeiten oder Körpergeweben als Markerpeptide zum diagnostischen Nachweis und für die Verlaufsprognose sowie die Verlaufskontrolle von Entzündungen und Infektionen, einschließlich Sepsis.

SEQUENCE LISTING

<110> B.R.A.H.M.S Aktiengesellschaft

<120> Verwendungen von Fragmenten der Carbamoylphosphat
      Synthetase 1 (CPS 1) für die Diagnose von
      Entzündungserkrankungen und Sepsis

<130> 3695 AS

<140>
<141>

<160> 6

<170> PatentIn Ver. 2.1

<210> 1
<211> 12
<212> PRT
<213> Primat (Pavian)

<400> 1
Gly Gln Asn Gln Pro Val Leu Asn Ile Thr Asn
  1               5                   10

<210> 2
<211> 6
<212> PRT
<213> Primat (Pavian)

<400> 2
Asn Gln Pro Val Leu Asn Ile
  1               5

<210> 3
<211> 13
<212> PRT
<213> Primat (Pavian)

<400> 3
Ala Gln Thr Ala His Ile Val Leu Glu Asp Gly Thr Lys
  1               5                   10

<210> 4
<211> 4
<212> PRT
<213> Primat (Pavian)

<400> 4
Thr Ala His Ile
  1

<210> 5
<211> 12
<212> PRT
<213> Primat (Pavian)

<400> 5
Ala Phe Ala Met Thr Asn Gln Ile Leu Val Glu Lys
1               5                   10

<210> 6
<211> 1500
<212> PRT
<213> Homo sapiens

<400> 6

## Beschreibung

Die vorliegende Erfindung betrifft Verwendungen von neuen löslichen Fragmenten des Enzyms Carbamoylphosphat Synthetase 1 (E.C. 6.3.4.16, nachfolgend stets abgekürzt CPS 1) für die medizinische Diagnostik von Entzündungserkrankungen und Sepsis. Sie beruht auf dem erstmaligen Nachweis des Auftretens von Fragmenten der CPS 1 in Lebergewebe von Primaten, bei denen experimentell durch Toxinverabreichunge eine Sepsis bzw. systemische Entzündung erzeugt worden war.

Die vorliegende Erfindung hat ihren Ursprung in intensiven Forschungsarbeiten der Anmelderin im Zusammenhang mit weiteren Verbesserungen der Diagnose und Therapie von Entzündungen und Infektionen, insbesondere von Entzündungen infektiöser Ätiologie und Sepsis.

Als Entzündungen (Inflammationen) werden ganz allgemein bestimmte physiologische Reaktionen eines Organismus auf verschiedenartige äußere Einwirkungen wie z.B. Verletzungen, Verbrennungen, Allergene, Infektionen durch Mikroorganismen wie Bakterien und Pilze und Viren, auf Fremdgewebe, die Abstoßungsreaktionen auslösen, oder auf bestimmte entzündungsauslösende endogene Zustände des Körpers, z.B. bei Autoimmunerkrankungen und Krebs, bezeichnet. Entzündungen können als harmlose, lokal begrenzte Reaktionen des Körpers auftreten, sind jedoch auch typische Merkmale zahlreicher ernster chronischer und akuter Erkrankungen von einzelnen Geweben, Organen, Organ- und Gewebsteilen.

Lokale Entzündungen sind dabei meist Teil der gesunden Immunreaktion des Körpers auf schädliche Einwirkungen, und damit Teil des lebenserhaltenden Abwehrmechanismus des Organismus. Wenn Entzündungen jedoch Teil einer fehlgeleiteten Reaktion des Körpers auf bestimmte endogene Vorgänge wie z.B. bei Autoimmunerkrankungen sind und/oder chronischer Natur sind, oder wenn sie systemische Ausmaße erreichen, wie beim systemischen Inflammationssyndrom (Systemic Inflammatory Response Syndrome, SIRS) oder bei einer auf infektiöse Ursachen zurückzuführenden schweren Sepsis, geraten die für Entzündungsreaktionen typischen physiologischen Vorgänge außer Kontrolle und werden zum eigentlichen, häufig lebensbedrohlichen Krankheitsgeschehen.

Es ist heute bekannt, dass die Entstehung und der Verlauf von entzündlichen Prozessen von einer beträchtlichen Anzahl von Substanzen, die überwiegend proteinischer bzw. peptidischer Natur sind, gesteuert werden bzw. von einem mehr oder weniger zeitlich begrenzten Auftreten bestimmter Biomoleküle begleitet sind. Zu den an Entzündungsreaktionen beteiligten endogenen Substanzen gehören insbesondere solche, die zu den Cytokinen, Mediatoren, vasoaktiven Substanzen, Akutphasenproteinen und/oder hormonellen Regulatoren gezählt werden können. Die Entzündungsreaktion stellt eine komplexe physiologische Reaktion dar, an der sowohl das Entzündungsgeschehen aktivierende endogene Substanzen (z.B. TNF-α) als auch desaktivierende Substanzen (z.B. Interleukin-10) beteiligt sind.

Bei systemischen Entzündungen wie im Falle einer Sepsis bzw. des septischen Schocks weiten sich die entzündungsspezifischen Reaktionskaskaden unkontrolliert auf den gesamten Körper aus und werden dabei, im Sinne einer überschießenden Immunantwort, lebensbedrohlich. Zu den gegenwärtigen Kenntnissen über das Auftreten und die möglichen Rolle einzelner Gruppen endogener entzündungsspezifischer Substanzen wird beispielsweise verwiesen auf A.Beishuizen, et al., "Endogenous Mediators in Sepsis and Septic Shock", Advances in Clinical Chemistry, Vol.33, 1999, 55-131; und C.Gabay, et al., "Acute Phase Proteins and Other Systemic Responses to Inflammation", The New England Journal of Medicine, Vol.340, No.6, 1999, 448-454. Da sich das Verständnis von Sepsis und verwandten systemischen entzündlichen Erkrankungen, und damit auch die anerkannten Definitionen, in den letzten Jahren gewandelt haben, wird außerdem verwiesen auf K.Reinhart, et al., "Sepsis und septischer Schock", in: Intensivmedizin, Georg Thieme Verlag, Stuttgart.New York, 2001, 756-760; wo eine moderne Definition des Sepsis-Begriffes gegeben wird. Im Rahmen der vorliegenden Anmeldung werden die Begriffe Sepsis bzw. entzündliche Erkrankungen in Anlehnung an die Definitionen verwendet, wie sie den genannten drei Literaturstellen entnommen werden können.

Während wenigstens im europäischen Raum die durch eine positive Blutkultur nachweisbare systemische bakterielle Infektion lange den Sepsisbegriff prägte, wird die Sepsis heute in erster Linie als systemische Entzündung verstanden, die infektiöse Ursachen hat, als Krankheitsgeschehen jedoch große Ähnlichkeiten mit systemischen Entzündungen aufweist, die durch andere Ursachen ausgelöst werden. Dem genannten Wandel des Sepsis-Verständnisses entsprechen Veränderungen der diagnostischen Ansätze. So wurde der direkte Nachweis bakterieller Erreger durch komplexe Überwachungen physiologischer Parameter und in jüngerer Zeit insbesondere auch durch den Nachweis bestimmter am Sepsisgeschehen bzw. am Entzündungsgeschehen beteiligter endogener Substanzen, d.h. spezifischer "Biomarker", ersetzt bzw. ergänzt.

Von der großen Zahl von Mediatoren und Akutphasenproteinen, von denen man weiß, dass sie an einem Entzündungsgeschehen beteiligt sind, eignen sich dabei für diagnostische Zwecke insbesondere solche, deren Auftreten sehr spezifisch für entzündliche Erkrankungen bzw. bestimmte Phasen entzündlicher Erkrankungen ist, deren Konzentrationen sich drastisch und diagnostisch signifikant verändern und die außerdem die für Routinebestimmungen erforderlichen Stabilitäten aufweisen und hohe Konzentrationswerte erreichen. Für diagnostische Zwecke steht dabei die zuverlässige Korrelation von Krankheitsgeschehen (Entzündung, Sepsis) mit dem jeweiligen Biomarker im Vordergrund, ohne dass dessen Rolle in der komplexen Kaskade der am Entzündungsgeschehen beteiligten endogenen Substanzen bekannt sein muss.

Eine derartige als Sepsis-Biomarker besonders geeignete endogene Substanz ist Procalcitonin. Procalcitonin ist ein Prohormon, dessen Serum-Konzentrationen unter den Bedingungen einer systemischen Entzündung infektiöser Ätiologie (Sepsis) sehr hohe Werte erreichen, während es bei Gesunden so gut wie nicht nachweisbar ist. Hohe Werte an Procalcitonin werden außerdem in einem relativ frühen Stadium einer Sepsis erreicht, so dass sich die Bestimmung von Procalcitonin auch zu Früherkennung einer Sepsis und zur frühen Unterscheidung einer infektiös bedingten Sepsis von schweren Entzündungen, die auf anderen Ursachen beruhen, eignet. Die Bestimmung von Procalcitonin als Sepsismarker ist Gegenstand der Veröffentlichung M.Assicot, et al., "High serum procalcitonin concentrations in patients with sepsis and infection", The Lancet, vol.341, No.8844, 1993, 515-518; und der Patente DE 42 27 454 C2 bzw. EP 0 656 121 B1 bzw. US 5,639,617. Auf die genannten Patente und in der genannten Veröffentlichung angeführten frühen Literaturstellen wird zur Ergänzung der vorliegenden Beschreibung ausdrücklich Bezug genommen.

Die Verfügbarkeit des Sepsismarkers Procalcitonin hat der Sepsisforschung starke Impulse gegeben, und es werden gegenwärtig intensive Anstrengungen unternommen, weitere Biomarker zu finden, die die Procalcitonin-Bestimmung ergänzen können und/oder zusätzliche Informationen für Zwecke der Feindiagnostik bzw. Differentialdiagnostik zu liefern vermögen. Erschwert wird die Suche nach potentiellen neuen Sepsis-Biomarkern allerdings dadurch, dass häufig noch sehr wenig oder nichts über die genaue Funktion bzw. über die genauen Gründe für das Auftreten bestimmter endogener Substanzen, die am Entzündungs- oder Sepsisgeschehen beteiligt sind, bekannt ist.

Die Ergebnisse der experimentellen Überprüfung eines fruchtbaren rein hypothetischen Ansatzes zur Ermittlung weiterer potentieller Sepsismarker finden sich in DE 198 47 690 A1 bzw. WO 00/22439. Dort wird gezeigt, dass bei Sepsis nicht nur die Konzentration des Prohormons Procalcitonin erhöht ist, sondern auch für andere Substanzen, die zu den Peptid-Prohormonen gerechnet werden können, signifikant erhöhte Konzentrationen beobachtet werden können. Während das beschriebene Phänomen gut dokumentiert ist, sind die Ursachen für den Anstieg der Konzentrationen von Prohormonen bei Sepsis weiterhin weitgehend ungeklärt.

In der vorliegenden Anmeldung wird ein Ergebnis eines anderen fruchtbaren, rein experimentellen Ansatzes für die Suche nach weiteren entzündungs- bzw. sepsisspezifischen Biomolekülen berichtet. Auch diese experimentellen Untersuchungen nehmen ihren Ausgang bei der Bestimmung von Procalcitin im Zusammenhang mit systemischen entzündlichen Reaktionen infektiöser Ätiologie. So war sehr früh beobachtet worden, dass bei Sepsis das Procalcitonin offensichtlich nicht auf die gleiche Weise gebildet wird, wie dann, wenn es Vorläufer für das Hormon Calcitonin ist. So wurden hohe Procaicitoninspiegel auch bei Patienten beobachtet, denen die Schilddrüse entfernt worden war. Deshalb kann die Schilddrüse nicht dasjenige Organ sein, in dem Procalcitonin bei Sepsis gebildet bzw. ausgeschüttet wird. In den Veröffentlichungen H.Redl, et al., "Procalcitonin release patterns in a baboon model of trauma and sepsis: Relationship to cytokines and neopterin", Crit Care Med 2000, Vol.28. No.11, 3659-3663; und H.Redl, et al., "Non-Human Primate Models of Sepsis", Sepsis 1998; 2:243-253; werden die Ergebnissen von experimentellen Untersuchungen berichtet, die der Klärung der Bildung von Procalcitonin bei Sepsis dienen sollten. In den genannten Arbeiten wird durch Endotoxinverabreichung an Primaten (Paviane) eine künstliche Sepsis erzeugt, und es wird bestimmt, bei welchen experimentell erzeugten Zuständen die höchsten Procalcitoninkonzentrationen im Blut erreicht werden. Eine Weiterentwicklung des in den genannten Arbeiten beschriebene Versuchstiermodells dient im Rahmen der vorliegenden Anmeldung dazu, neue endogene sepsisspezifische Biomarker von peptischer bzw. proteinischer Natur zu ermitteln, deren Auftreten für Sepsis oder bestimmte Formen von Sepsis charakteristisch ist und die daher eine spezifische Sepsisdiagnose ermöglichen. Das Primatenmodell wurde dabei aufgrund der sehr großen Ähnlichkeit der Physiologie von Primaten und Menschen und der hohen Kreuzreaktivität mit vielen therapeutischen und diagnostischen humanen Reagenzien gewählt.

Da die bei Entzündungen gebildeten endogenen Substanzen Teil der komplexen Reaktionskaskade des Körpers sind, sind derartige Substanzen außerdem nicht nur von diagnostischem Interesse, sondern es wird gegenwärtig auch mit erheblichem Aufwand versucht, durch Beeinflussung der Entstehung und/oder der Konzentration einzelner derartiger Substanzen therapeutisch in das Entzündungsgeschehen einzugreifen, um die zum Beispiel bei Sepsis beobachtete systemische Ausbreitung der Entzündung möglichst frühzeitig zu stoppen. In diesem Sinne sind nachweislich am Entzündungsgeschehen beteiligte endogene Substanzen auch als potentielle therapeutische Targets anzusehen. Trotz der bisherigen eher enttäuschenden Ergebnisse derartiger therapeutischer Ansätze besteht weiterhin ein hohes Interesse daran, bisher im entsprechenden Zusammenhang nicht beschriebene, möglichst entzündungs- bzw. sepsisspezifische endogene Biomoleküle zu identifizieren, die auch als therapeutische Targets neue Erfolgsaussichten für die therapeutische Sepsiskontrolle eröffnen.

Die vorliegenden Erfindung beruht darauf, dass sich in Primaten und Menschen bei infektiös bedingten Entzündungen Fragmente des Enzyms Carbamoylphosphat Synthetase (CPS 1) nachweisen lassen, und zwar im Unterschied zu unbehandelten Kontrollindividuen bzw. Gesunden, bei denen diese nicht gefunden werden, was derartige CPS 1-Fragmente für die Entzündungsdiagnostik/Sepsisdiagnosik geeignet macht.

Die Verwendungen in der Diagnostik, die sich aufgrund des erstmals nachgewiesenen Auftretens von Fragmenten von CPS 1 bei der experimentellen Simulation von Entzündungen bzw. Sepsis eröffnen, werden in allgemeiner Form in den Ansprüchen 1 bis 4 beansprucht.

Die Ansprüche 5 bis 14 betreffen die sich aus den neuen Erkenntnissen ergebenden Varianten diagnostischer Verfahren

Wie nachfolgend im experimentellen Teil noch näher ausgeführt wird, beruht die Erfindung darauf, dass nach experimenteller Auslösung einer künstlichen Sepsis in Pavianen durch Endotoxinverabreichung (LPS aus Salmonella Typhimurium) und 2D-gelektrophoretischer Aufarbeitung von Lebergewebe der behandelten Tiere eine Gruppe von nur bei den behandelten Tieren identifizierbaren benachbarten Proteinspots gefunden werden konnte. Die den Spots entsprechenden proteinischen Produkte mit (gelektrophoretisch bestimmten) molaren Massen von ca. 68 kDa, 69 kDa und 70 kDa ± 3 kDa wurden aus dem Elektrophoresegel isoliert, massenspektrometrisch untersucht und als lösliche Fragmente von CPS 1 identifiziert.

Und zwar wurden bei der massenspektrometischen Analyse aller drei aus dem Gel isolierten Proteinspots durch Tandem-Massenspektrometrie aus allen drei Proteinspots kurze, teilweise identische Teilpeptide ("Tags") identifiziert, die sich in identischer Form in der Sequenz der humanen CPS 1 (SEQ ID NO:6) wiederfanden, wobei die konkret identifizierten Peptide in Richtung des C-Terminus der CPS 1 Aminosäuren bis Position 624 umfaßten.

Aufgrund der Identität der identifizierten massenspektrometrischen Fragmente mit Teilsequenzen aus dem N-terminalen Teil der CPS 1 ist die Identifizierung der untersuchten Proteinspotss als CPS 1-Fragmente nach anerkannten Kriterien als eindeutig anzusehen.

Die Identifizierung der nur nach Sepsis- bzw. Entzündungsauslösung in dem Pavian-Lebergewebe gefundenen Proteine als Fragmente aus den N-terminalen Teil der CPS 1 ist von hohem wissenschaftlichen, diagnostischen und therapeutischen Interesse.

Für die medizinische Diagnostik spielten CPS 1-Fragmente bisher noch keine praktische Rolle. Das Enzym CPS 1 ((E.C. 6.3.4.16) selbst ist jedoch seit langem gut bekannt. Es katalysiert die Umwandlung von Ammoniak, Bicarbonat und 2 ATP unter Bildung von Carbamoylphosphat im ersten Schritt des Harnstoffzyklus. Es spielt dabei auch eine Rolle bei der Biosynthese von Arginin, das seinerseits ein Substrat für die Biosynthese von NO, z.B. bei einem Endotoxin-Schock, ist (vgl. Shoko Tabuchi et al., Regulation of Genes for Inducible Nitric Oxide Synthase and Urea Cycle Enzymes in Rat Liver in Endotoxin Shock, Biochemical and Biophysical Research Communications **268,** 221-224 (2000)). CPS 1 ist von dem cytosolischen Enzym CPS 2 (E.C. 2.7.2.5.) zu unterscheiden, das ebenfalls eine Rolle im Harnstoffzyklus spielt, jedoch das Substrat Glutamin verarbeitet. Es ist bekannt, dass CPS 1 in Mitochondrien lokalisiert ist und im Lebergewebe in dieser Form in großen Mengen (es macht von 2-6% des Leber-Gesamtproteins aus) vorkommt. Seine Aminosäuresequenz (SEQ ID NO:6) und genetische Lokalisierung ist seit langem bekannt (vgl. Haraguchit Y. et al, Cloning and sequence of a cDNA encoding human carbamyl phosphate synthetase I: molecular analysis of hyperammonemia, Gene 1991, Nov. 1; 107 (2):-335-340). Zu seiner physiologischen Rolle kann verwiesen werden auf Reviewartikel wie z.B. H.M.Holder et al., Carbamoyl phosphate synthetase: an amazing biochemical odyssey from substrate to product, CMLS, Cell.Mol.Life Sci. **56** (1999) 507-522 und die darin referierte Literatur, sowie die Einleitung der Veröffentlichung Mikiko Ozaki et al., Enzyme-Linked Immunosorbent Assay of Carbamoylphosphate Synthetase I: Plasma Enzyme in Rat Experimental Hepatitis and Its Clearance, Enzyme Protein 1994, 95:48:213-221.

Gemäß Shoko Tabuchi et al., loc.cit. wird in Rattenlebern bei einem künstlichen Endotoxin-Schock (LPS) keine Erhöhung des Enzyms (Proteins) beobachtet. Gemäß Li Yin et al., Participation of different cell types in the restitutive response of the rat liver to periportal injury induced by allyl alcohol, Journal of Hepatology 1999, 31:497-507, läßt sich bei einer Leberschädigung durch Allylakohol bei histologischen Untersuchungen nach drei Tagen in allen Hepatocyten eine Steigerung der CPS 1-Expression beobachten.

Es urde ferner festgestellt, dass sich bei einer durch Verabreichung von Galactosamin experimentell erzeugten akuten Hepatitis bei Ratten im Plasma der Ratten eine stark erhöhte immunologische CPS 1-Aktivität findet (nachgewiesen mit einem ELISA mit anti-Ratten CPS 1 IgG aus Kaninchen), und zwar 24-48 h nach der Behandlung mit dem Hepatitis-auslösenden Galactosamin. Im Rattenplasma wurden während der akuten Hepatitis auch zunehmend CPS 1-Fragmente mit molaren Massen von ca. 140 und 125 kDa ohne sonstige nähere Charakterisierung (Sequenzzuodnung) erkennbar, während bei einer begleitenden Immunoblotting-Analyse in humanen Autopsie-Proben keine CPS 1-Fragmente mit CPS 1-Immunreaktivität beobachtet werden konnten (Mikiko Ozaki et al., loc. cit.).

Eine Eignung von löslichen CPS 1-Fragmenten, insbesondere von Fragmenten mit molaren Massen von 68-70 kDa ± 3 kDa aus dem N-terminalen Teil der CPS 1, als Biomarker für die Diagnose von Entzündungen und Sepsis bei Menschen, der in humanem Serum oder Plasma bestimmt werden kann, läßt sich aus diesen Befunden nicht ableiten.

Aufgrund der nachgewiesenen verstärkten Bildung von Fragmenten der humanen CPS 1 bei Pavianen mit einer experimentell erzeugten Sepsis, und zwar im Gegensatz zu unbehandelten bzw. gesunden Tieren, in deren Lebergewebe trotz identischer Aufarbeitung und Lagerung keine derartigen Fragmente nachweisbar waren, eignen sich derartige Fragmente für diagnostische Zwecke. Werden für den Nachweis nach an sich bekannten immundiagnostischen Verfahren derartige Fragmente als Reagenzien oder zur Erzeugung bestimmter spezifischer Antikörper benötigt, können die Fragment nach Verfahren, die inzwischen zum Stand der Technik gehören, synthetisch oder gentechnologisch als Rekombinationsprodukte hergestellt werden.

Ferner können die CPS 1-Fragmente nach bekannten Verfahren des modernen Standes der Technik auch zur Erzeugung spezifischer polyklonaler und insbesondere monoklonaler Antikörper verwendet werden, die als Hilfsmittel für die diagnostische Bestimmung der erfindungsgemäßen Peptide und/oder auch als potentielle therapeutische Mittel geeignet sind. Die Erzeugung geeigneter monoklonaler Antikörper gegen bekannte Peptid-Teilsequenzen gehört heute zum allgemeinen Stand der Technik.

Bei der Bestimmung der CPS 1-Fragmente in Patientenseren davon kann grundsätzlich so vorgegangen werden, wie das z.B. für die selektive Procalcitoninbestimmung beschrieben ist in P.P.Ghillani, et al., "Monoclonal antipeptide antibodies as tools to dissect closely related gene products", The Journal of Immunology, vol. 141, No.9, 1988, 3156-3163; und P.P.Ghillani, et al., "Identification and Measurement of Calcitonin Precursors in Serum of Patients with Malignant Diseases", Cancer research, vol.49, No.23, 1989, 6845-6851; wobei ausdrücklich ergänzend auch auf die dort beschriebenen Immunisierungstechniken verwiesen wird, die eine Möglichkeit für die Gewinnung von monoklonalen Antikörpern auch gegen Teilsequenzen der CPS 1 darstellen. Variationen der beschriebenen Techniken und/oder weitere Immunisierungstechniken kann der Fachmann einschlägigen Standardwerken und Veröffentlichungen entnehmen und sinngemäß anwenden.

Als Verwendung im Sinne der vorliegenden Anmeldung ist auch eine Verwendung von löslichen CPS 1-Fragmenten als Bestandteil (Reagens) eines Assaykits anzusehen, oder eine Verwendung zur Erzeugung von Assaykomponenten, z.B. poly- oder monoklonalen Antikörpern.

Dabei ist auch ausdrücklich die Erzeugung von CPS 1-Antikörpern unter Anwendung von Techniken der direkten genetischen Immunisierung mit DNA zu erwähnen. Es liegt im Rahmen der vorliegenden Erfindung, zur Immunisierung z.B. eine cDNA der gewünschten CPS 1-Fragmente zu verwenden, da es sich in der Vergangenheit gezeigt hat, dass durch derartige Immunisierungstechniken das Spektrum der gewinnbaren Antikörper erweitert werden kann.

Es ist zusätzlich ausdrücklich darauf hinzuweisen, dass es bei der erfindungsgemäßen Bestimmung von CPS 1-Fragmenten aus dem N-terminalen Teil der CPS 1-Sequenz je nach Assaydesign auch dazu kommen kann, dass ggf. gleichzeitig in der biologischen Flüssigkeit vorhandene längere lösliche CPS 1-Bruchstücke, die diese Fragmente enthalten, oder auch in löslicher Form vorliegende Formen der vollständigen CPS 1 (die normalerweise in den Mitochondrien lokalisiert ist) mitbestimmt werden. Auch derartige Verfahren sind, im Sinne der vorliegenden Erfindung, als erfindungsgemäße Verfahren zur Bestimmung von CPS 1-Fragmenten anzusehen.

Lösliche Formen von CPS 1 gemäß SEQ ID NO:6 oder lösliche Teilpeptide davon, z.B. solche, die eine der Teilsequenzen von SEQ ID NO:1 bis SEQ ID NO:5 und/oder andere Teilsequenzen aus dem N-Terminus von CPS 1 enthalten oder daraus bestehen, können somit aufgrund der vorliegenden Ergebnisse als spezifische Markerpeptide (Biomarker) zum diagnostischen Nachweis und zur Verlaufskontrolle von Entzündungen und Infektionen (insbesondere, wie Procalcitonin, auch von systemischen Infektionen vom Sepsistyp) dienen.

An Stelle der Bestimmung der CPS 1-Bruchstücke oder ggf. posttranslational modifizierter Formen davon soll für diagnostische Zwecke ggf. auch eine Bestimmung der zugehörigen mRNA nicht ausgeschlossen sein. Die CPS 1-Bestimmung kann für diagnostische Zwecke u.U. auch indirekt als Bestimmung einer Enzymaktivität, die der (Rest-)Aktivität der Fragmente entspricht, erfolgen.

Es ist ferner möglich, die Bestimmung von CPS 1-Fragmenten als Prognosemarker und Marker für die Überwachung der Krankheitsverlaufs von Entzündungen, insbesondere systemischen Entzündungen, und Sepsis im Rahmen einer Kombinationsmessung mit anderen Markern durchzuführen.

Neben einer Kombination mit einer Procalcitoninmessung kommt insbesondere eine Kombination der Messung von CPS 1 mit der Bestimmung anderer Marker für Sepsis und systemische Entzündungen in Betracht, und zwar insbesondere mit CA 19-9, CA 125, S100B oder an der Regulierung von Entzündungen beteiligten S100A-Proteinen, oder mit der Bestimmung der in den älteren, nachfolgend genannten unveröffentlichten deutschen Patentanmeldungen der Anmelderin beschriebenen neuartigen Sepsismarker Inflammin (DE 101 19 804.3) und CHP (DE 101 31 922.3), des Proteins LASP-1 und/oder mit der Bestimmung von löslichen Cytokeratinfragmenten, insbesondere der neu aufgefundenen löslichen Cytokeratin-1-Fragmente (sCY1F;; DE 101 30 985.6) sowie der bekannten Tumormarker CYFRA-21 oder TPS und/oder eines oder mehrerer der o.g. Prohormone. Auch eine gleichzeitige Bestimmung des bekannten Entzündungsparameters C-reaktives Protein (CRP) kann vorgesehen sein. Aufgrund der in dieser und den verwandten Anmeldungen der Anmelderin beschriebenen neuen Ergebnisse ist für die Sepsis-Feindiagnose außerdem generell eine Kombination mit Messungen von bekannten oder noch aufzufindenden Biomolekülen in Betracht zu ziehen, die als gewebe- bzw. organspezifische Entzündungsmarker geeignet sind.

Der Inhalt der genannten älteren Anmeldungen der Anmelderin ist durch die ausdrückliche Bezugnahme auf diese Anmeldungen als Teil der Offenbarung der vorliegenden Anmeldung anzusehen.

Nachfolgend wird die Auffindung und Identifizierung der CPS 1-Fragmente in näheren Einzelheiten geschildert, wobei auf das beigefügte Sequenzprotokoll bezug genommen wird. Die Figur zeigt:

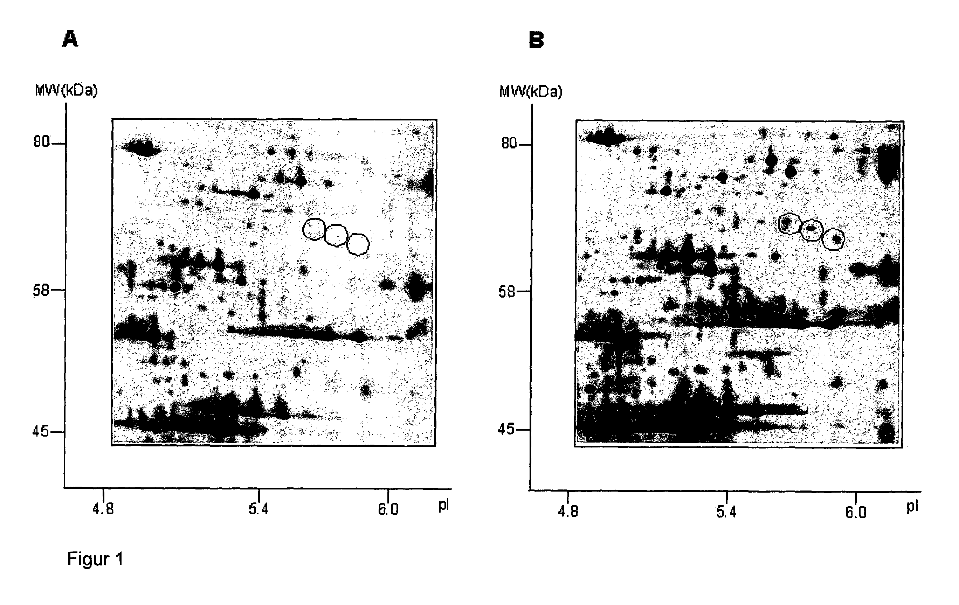

- Fig. 1: Ansichten von 2D-Elektrophoresegelen, die einen Vergleich der Spotmuster von cytoplasmatischen Leberzellprotein eines gesunden Pavians (A) mit den Leberzellproteinen eines Pavians 5h nach einer durch LPS-Verabreichung induzierten Sepsis (B) ermöglichen. Der Pfeil zeigt die Positionen der drei erfindungsgemäßen sepsisspezifischen Produkte (CPS 1-Fragmente) an, die in Darstellung (B) durch einen Kreis hervorgehoben sind;

### 1. Infektionssimmulation durch Endotoxinverabreichung im Tiermodell (Paviane).

In Anlehnung an die mit Pavianen durchgeführten Versuche zur Stimulierung der Procalcitonin-Ausschüttung durch Endotoxininjektionen (vgl.H.Redl, et al., "Procalcitonin release patterns in a baboon model of trauma and sepsis: Relationship to cytokines and neopterin", Crit Care Med 2000, Vol.28. No.11, 3659-3663; H.Redl, et al., "Non-Human Primate Models of Sepsis", in: Sepsis 1998; 2:243-253) wurden Pavianen (männlich, ca. 2 Jahre alt, 27 bis 29 kg schwer) jeweils 100 µg LPS (Lipopolysaccharid aus Salmonella Typhimurium, Bezugsquelle: Sigma) pro kg Körpergewicht intravenös verabreicht. 5 bis 5,5 h nach der Injektion wurden die Tiere durch intravenöse Verabreichung von 10 ml Doletal getötet. Innerhalb von 60 min nach ihrem Exitus wurden sämtliche Organe/Gewebe präpariert und durch Einfrieren in flüssigem Stickstoff stabilisiert.

Bei der weiteren Verarbeitung wurden Proben der einzelnen tiefgefrorenen Gewebe (1g) unter Stickstoffkühlung mit 1,5 ml Puffer A (50mM Tris/HCl, pH 7,1, 100mM KCl, 20% Glycerol) versetzt und in einem Porzellanmörser zu einem Mehl pulverisiert (vgl. J.Klose, "Fractionated Extraction of Total Tissue Proteins from Mouse and Human for 2-D Electrophoresis", in: Methods in Molecular Biology, Vol.112: 2-D Proteome Analysis Protocols, Humana Press Inc., Totowa, NJ). Nach einer anschließenden 1-stündigen Zentrifugation bei 100.000 g und +4°C wurde der erhaltene Überstand gewonnen und bis zur weiteren Verarbeitung bei -80°C gelagert.

Weil Versuche mit den wie oben gewonnenen Proben ergeben hatten, dass die größten Procalcitoninmengen in Lebergewebe behandelter Tiere gefunden wird, wurde für die Suche nach neuen sepsisspezifischen Biomarkern mit Proteinextrakten aus der Pavianleber gearbeitet.

### 2. Proteomanalyse unter Verwendung cytoplasmatischer Leberzellproteine von Pavianen.

Cytoplasmatische Leberzellproteinextrakte von einerseits gesunden Pavianen (Kontrolle) und andererseits Pavianen, denen LPS injiziert worden war, wurden im Rahmen einer Proteomanalyse verwendet. Bei der einleitenden analytischen 2D-Gelelektrophorese wurde Leberextrakt, 100 µg Protein enthaltend, auf 9M Harnstoff, 70 mM DTT, 2% Ampholyt pH 2-4 eingestellt und dann mittels analytischer 2D-Gelelektrophorese aufgetrennt, wie in J.Klose, et al., "Two-dimensional electrophoresis of proteins: An updated protocol and implications for a functional analysis of the genome", Electrophoresis 1995, 16, 1034-1059; beschrieben ist. Die Sichtbarmachung der Proteine im 2D-Elektrophoresegel erfolgte mittels Silverstaining (vgl. J.Heukeshoven, et al., "Improved silver staining procedure for fast staining in Phast-System Development Unit. I. Staining of sodium dodecyl gels", Electrophoresis 1988, 9, 28-32).

Zur Auswertung wurden die Proteinspotmuster der Proben unbehandelter Tieren mit den Proteinspotmustern verglichen, die aus Lebergewebeproben behandelter Tiere resultierten. Substanzen, die bei keiner Kontrollprobe, aber bei allen behandelten Tieren zusätzlich auftraten, wurden für weitere analytische Untersuchungen selektiert. Fig. 1 zeigt einen Vergleich der 2D-Elektrophoresegele für eine Kontrollprobe (A) und eine Probe eines behandelten Tieres (B), wobei drei zusätzliche Proteinspots in (B) mit molaren Massen von ca. 68 kDa, 69 kDA und 70 kDa (± 3 kDa) und isoelektrischen Punkten von ca. 6,0, 5,8 bzw. 5,6 durch einen Pfeil und einen Kreis hervorgehoben sind.

Die im Proteinspotmuster der analytischen 2D-Gelelektrophorese identifizierten neuen spezifischen Proteine wurden dann anschließend mittels präparativer 2D-Gelelektrophorese unter Einsatz von 350 µg Protein präpariert (vgl. wiederum (10). Bei der präparativen 2D-Gelelektrophorese erfolgte die Färbung mittels Coomassie Brilliant Blue G250 (vgl. V.Neuhoff, et al., "Improved staining of proteins in polyacrylamide gels including isoelectric focusing gels with clear background at nanogram sensitivity using Coomassie Brilliant Blue G-250 and R-250", Electrophoresis 1988, 9, 255-262).

Die für die weitere Analyse vorselektierten Proteinspots wurden aus dem Gel ausgeschnitten.

Die Proteinspots wurden jeweils unter Anwendung der Methode, die in A.Otto, et al., "Identification of human myocardial proteins separated by two-dimensional electrophoresis using an effective sample preparation for mass spectrometry", Electrophoresis 1996, 17, 1643-1650; beschrieben ist, trypsinverdaut und anschließend massenspektroskopisch analysiert und zwar unter Anwendung massenspektrometrischer Untersuchungen, wie sie z.B. in G.Neubauer, et al., "Mass spectrometry and EST-database searching allows characterization of the multi-protein spliceosome complex", in: nature genetics vol. 20, 1998, 46-50; J.Lingner, et al., "Reverse Transcriptase Motifs in the Catalytic Subunit of Telomerase", in: Science, Vol.276, 1997, 561-567; M.Mann, et al., "Use of mass spectrometry-derived data to annotate nucleotide and protein sequence databases", in: TRENDS in Biochemical Sciences, Vol.26, 1, 2001, 54-61; beschrieben und diskutiert werden.

Dabei wurden Fragmente des Trypsinverdaus aller drei Proteinspots nach einer ESI (ElectroSprayIonisierung) auch einer Tandem-Massenspektrometrie unterzogen. Es wurde ein Q-TOF-Massenspektrometer mit einer sog. nanoflow-Z-Spraylonenquelle der Firma Micromass, UK, verwendet. Dabei wurde entsprechend der Arbeitsanleitung des Geräteherstellers gearbeitet.

### 3. Identifizierung von CPS 1-Fragmenten

Wie in den Figuren 1(A) und 1(B) gezeigt ist, finden sich in Leberzellextrakten von Pavianen, denen eine LPS-Injektion verabreicht worden war, u.a. drei neue Proteinspots, für die aufgrund der Gelektrophoresedaten im Vergleich mit Markersubstanzen mit bekanntem Molekulargewicht Molekulargewichte von ca. 68 kDa, 69 kDa und 70 kDa (± 3 kDa) abgeschätzt wurden, während aus der relativen Position der Proteine aus der ersten Dimension zugehörige isoelektrische Punkte von etwa 6,0, 5,8 bzw. 5,6 ermittelt wurden, d.h. isoelektrische Punkte im Bereich von ca. 5,5 bis 6,1.

Diese Proteine wurden wie oben erläutert massenspektrometrisch analysiert.

Aus den "Mutterspektren" der drei trypsinverdauten Poteine wurden jeweils einzelne Fragmente ("Tags") durch Tandem-Massenspektroskopie identifiziert. Die für diese Fragmente erhaltenen Massenspektren konnten auf an sich bekannte Weise rechnerisch ausgewertet werden und lieferten die folgenden Ergebnisse (massenspektrometrisch ist keine Unterscheidung zwischen den Aminosäuren Leucin (L) und Isoleucin (I) sowie den Aminosäuren Lysin (K) und Glutamin (Q) möglich; die nachfolgenden Sequenzen berücksichtigen daher bereits die Zuordnung zum bekannten Spektrum der vollständigen CPS 1 gemäß SEQ ID NO:6):
Proteinspot bei 70 kDa (± 3 kDa): Fragment 70/1:     GQNQPVLNITN     (SEQ ID NO:1) Fragment 70/2:     NQPVLNI     (SEQ ID NO:2) Fragment 70/3:      AQTAHIVLEDGTK  (SEQ ID NO:3) Proteinspot bei 69 kDa (± 3 kDa): Fragment 69/1:     GQNQPVLNITN     (SEQ ID NO:1) Fragment 69/2:      TAHI                (SEQ ID NO:4) Proteinspot bei 68 kDa (± 3 kDa):

Fragment 68/1:     NQPVLNI     (SEQ ID NO:2)

Fragment 68/2:     AFAMTNQILVEK   (SEQ ID NO:5).

Die obigen Teilsequenzen gemäß SEQ ID NO:1 bis SEQ ID NO:5 konnten als Teilsequenzen der unter NiceProt View of SWISS PROT: P31327 zu findenden Sequenz der humanen CPS 1 mit einer Aminosäurekette einer Länge von 1500 Aminosäuren und einer zugehörigen rechnerischen molaren Masse (ohne Berücksichtigung evtl. posttranslationaler Modifizierungen) von 164,939 kDa (SEQ ID NO:6) identifiziert werden. Es ergab sich die folgenden Zuordnung der Teilpeptide:

SEQ ID NO:1    Aminosäuren 317 - 327

SEQ ID NO:2    Aminosäuren 319 - 325

SEQ ID NO:3    Aminosäuren  43 - 55

SEQ ID NO:4    Aminosäuren  45 - 48

SEQ ID NO:5    Aminosäuren 613 - 624

Die gefundenen Aminosäuren überspannen einen Abschnitt von Aminosäure 43 bis Aminosäure 624, d.h. einen wesentlichen Teil des aminoterminalen Teils der CPS 1.

Es ist ergänzend darauf hinzuweisen, dass sich die gefundenen Sequenzen nicht dem verwandten cytosolischen Enzym CPS 2 zuordnen ließen.

## Patentansprüche

1. Verwendung von Fragmenten des N-terminalen Teils der Carbamoylphosphat Synthetase (CPS 1) als Markerpeptide zum diagnostischen Nachweis und für die Verlaufsprognose sowie die Verlaufskontrolle von Entzündungen und Infektionen.

2. Verwendung von CPS 1-Fragmenten nach Anspruch 1 im Rahmen der differentialdiagnostischen Früherkennung und Erkennung für die Verlaufsprognose, die Beurteilung des Schweregrads und für die therapiebegleitende Verlaufsbeurteilung von Sepsis und schweren Infektionen, insbesondere sepsisähnlichen systemischen Infektionen, durch Bestimmung des Auftretens und/oder der Menge von löslichen CPS 1-Fragmenten in einer biologischen Flüssigkeit eines Patienten.

3. Verwendung nach einem der Ansprüche 1 oder 2 von CPS 1-Fragmenten aus einem die Aminosäuren 1 bis ca. 630 der vollständigen CPS 1-Sequenz (SEQ ID NO:6) umfassenden N-terminalen Teil der CPS 1-Sequenz.

4. Verwendung nach einem der Ansprüche 1 bis 3, **dadurch gekennzeichnet, dass** die CPS 1-Fragmente ausgewählt sind aus Fragmenten mit einer gelelektrophoretisch bestimmten molaren Masse von 68 bis 70 kDa ± 3 kDa und isoelektrischen Punkten in einem Bereich von 5,5 bis 6,1.

5. Verfahren zur differentialdiagnostischen Früherkennung und Erkennung, für die Verlaufsprognose und die Beurteilung des Schweregrads und zur therapiebegleitenden Verlaufsbeurteilung von Sepsis und schweren Infektionen, insbesondere sepsisähnlichen systemischen Infektionen, **dadurch gekennzeichnet, dass** man die Anwesenheit und/oder Menge von Fragmenten aus dem N-terminalen Teil der CPS 1 in einer biologischen Flüssigkeit eines Patienten bestimmt und aus dem Nachweis und/oder der Menge mindestens eines der bestimmten Fragmente Schlüsse hinsichtlich des Vorliegens, des zu erwartenden Verlaufs, des Schweregrads oder des Erfolgs eines Therapie einer Sepsis oder Infektion zieht.

6. Verfahren nach Anspruch 5, **dadurch gekennzeichnet, dass** die bestimmten CPS 1-Fragmente Fragmente aus einem die Aminosäuren 1 bis ca. 630 der vollständigen CPS 1-Sequenz (SEQ ID NO:6) umfassenden N-terminalen Teil der CPS 1 sind.

7. Verfahren nach Anspruch 5 oder 6, **dadurch gekennzeichnet, dass** die bestimmten CPS 1-Fragmente Fragmente mit einer gelelektrophoretisch bestimmten molaren Masse von 68 bis 70 kDa ± 3 kDa und isoelektrischen Punkten in einem Bereich von 5,5 bis 6,1 sind.

8. Verfahren nach einem der Ansprüche 5 bis 7, **dadurch gekennzeichnet, dass** die bestimmten CPS 1-Fragmente solche Fragmente sind, die wenigstens 2 Aminosäure-Teilsequenzen gemäß SEQ ID NO:1, SEQ ID NO:2, SEQ ID NO:3, SEQ ID NO:4 und/oder SEQ ID NO:5 enthalten.

9. Verfahren nach einem der Ansprüche 5 bis 8, **dadurch gekennzeichnet, dass** es ein immundiagnostisches Bestimmungsverfahren ist.

10. Verfahren nach einem der Ansprüche 5 bis 8, **dadurch gekennzeichnet, dass** Bestimmung der CPS 1-Fragmente indirekt als Bestimmung des zugehörigen CPS 1-mRNA-Fragments erfolgt.

11. Verfahren nach einem der Ansprüche 5 bis 10, **dadurch gekennzeichnet, dass** es im Rahmen einer Multiparameter-Bestimmung durchgeführt wird, bei der gleichzeitig mindestens ein weiterer Sepsisparameter bestimmt wird und ein Messergebnis in Form eines Satzes von mindestens zwei Messgrößen gewonnen wird, der zur Sepsis-Feindiagnostik ausgewertet wird.

12. Verfahren nach Anspruch 11, **dadurch gekennzeichnet, dass** im Rahmen der Multiparameter-Bestimmung neben wenigstens einem CPS 1-Fragment wenigstens ein weiterer Parameter bestimmt wird, der ausgewählt ist aus der Gruppe, die besteht aus Procalcitonin, CA 19-9, CA 125, S100B, S100A-Proteinen, löslichen Cytokeratin-Fragmenten, insbesondere CYFRA 21, TPS und/oder löslichen Cytokeratin-1-Fragmenten (sCY1F), den Peptiden Inflammin, CHP, LASP-1 sowie Peptid-Prohormonen und dem C-reaktiven Protein (CRP).

13. Verfahren nach Anspruch 11 oder 12, **dadurch gekennzeichnet, dass** die Multiparameter-Bestimmung als Simultanbestimmung mittels einer Chiptechnologie-Messvorrichtung oder einer immunchromatographischen Messvorrichtung erfolgt.

14. Verfahren nach Anspruch 13, **dadurch gekennzeichnet, dass** die Auswertung des mit der Messvorrichtung gewonnenen komplexen Messergebnisses mit Hilfe eines Computerprogramms erfolgt.
